# EUROPEAN PATENT APPLICATION

(11) **EP 4 404 165 A1**
(43) Date of publication of application: **24.07.2024**
(21) Application number: 21957583.4
(22) Date of filing: 17.09.2021
(51) Int. Cl.: G08B 21/22, G08B 25/04

(54) **BEHAVIOR MONITORING SYSTEM AND BEHAVIOR MONITORING METHOD**

(71) Applicant: Fuji Corporation, Chiryu-shi, Aichi 472-8686 (JP)
(72) Inventor: SHIMIZU, Satoshi, Chiryu-shi, Aichi 472-8686 (JP); KITO, Shuichiro, Chiryu-shi, Aichi 472-8686 (JP)
(74) Representative: Grünecker Patent- und Rechtsanwälte PartG mbB
(86) International application number: PCT/JP2021/034435
(87) International publication number: WO 2023/042402

(57) **Abstract**

A behavior monitoring system includes a detection section, a storage section, and a processing section. The detection section is installed in a house and includes multiple occupancy sensors configured to detect a monitoring target person in a non-contact manner in detection ranges different with each other. The processing section is configured to acquire a distance between two points among the multiple occupancy sensors in advance to store the distance in the storage section. The processing section is configured to store reaction time as reaction data in the storage section in a case where any of the multiple occupancy sensors reacts, and calculate a movement distance of the monitoring target person per unit time based on data stored in the storage section.

## Description

### Technical Field

The present description discloses a behavior monitoring system and a behavior monitoring method.

### Background Art

Conventionally, as this type of behavior monitoring system, there has been proposed a system in which a management center remotely diagnoses a health state of an elderly person who lives alone (for example, refer to Patent Literature 1). In this system, multiple different pieces of life data related to an elderly person who lives in a house are measured and transmitted to a management center. The multiple different pieces of life data include a standard deviation of the number of times of mat operation of a tread force mat provided at an entry door of a bedroom, a lavatory, an entrance, a sitting room, a cooking area, a bathroom, a washroom, or the like of a house, an information value and a correction value of a sleep time, a mat operation frequency, a moving speed, and the number of times visiting the toilet. The management center gains each piece of life data on a daily basis, obtains an average value and a standard deviation for each piece of life data in a unit period, and obtains a unique vector indicating relations between multiple pieces of life data in the unit period. When measurement values of multiple pieces of life data on a daily basis are input, the management center calculates standardized data of each piece of life data by using the average value and the standard deviation for each piece of life data in a unit period, and obtains a life pattern (behavior pattern) on a daily basis with the standardized data of each piece of life data and two unique vector components having a large influence on a life rhythm of the elderly person among unique vectors indicating relations between the pieces of life data. Then, the management center determines a health state of the elderly person by monitoring a comprehensive gap between the obtained life pattern and a life pattern in the past unit period.

### Patent Literature

Patent Literature 1: JP-A-2003-275181

### Summary of the Invention

### Technical Problem

However, in the above-described system, since the tread force mat is used for the measurement of the life data, places where the tread force mat can be installed are limited to an entry door of a room, and there is a case where the places are insufficient for monitoring a behavior of a monitoring target person. In addition, there is a possibility that the tread force mat interferes with walking of an elderly person with weakened walking capability.

A main object of the present disclosure is to enable a behavior of a monitoring target person to be appropriately monitored with a simple configuration without interfering with the behavior of the monitoring target person.

### Solution to Problem

The present disclosure employs the following means in order to achieve the main object described above.

A behavior monitoring system of the present disclosure is summarized as a behavior monitoring system configured to monitor a behavior of a monitoring target person living in a house, the behavior monitoring system including a detection section installed in the house and including multiple occupancy sensors configured to detect the monitoring target person in a non-contact manner in detection ranges different with each other, a storage section configured to store data; and a processing section configured to acquire a distance between two points among the multiple occupancy sensors in advance to store the distance in the storage section, store reaction time as reaction data in the storage section in a case where any of the multiple occupancy sensors reacts, and calculate a movement distance of the monitoring target person per unit time based on data stored in the storage section.

The behavior monitoring system of the present disclosure includes the detection section, the storage section, and the processing section. The detection section is installed in the house and includes the multiple occupancy sensors configured to detect the monitoring target person in a non-contact manner in the detection ranges different with each other. The processing section acquires the distance between two points among the multiple occupancy sensors in advance and stores the distance in the storage section. Then, the processing section stores the reaction time as the reaction data in the storage section in a case where any of the multiple occupancy sensors reacts, and calculate the movement distance of the monitoring target person per unit time based on the data stored in the storage section. Since the detection section is configured with the occupancy sensor capable of detecting the monitoring target person in a non-contact manner, the detection section has a degree of freedom of installation and can be installed at a position that does not interfere with the behavior of the monitoring target person. Further, since the movement distance of the monitoring target person per unit time is calculated based on reaction data (reaction time) of each occupancy sensor, the behavior of the monitoring target person can be monitored appropriately with a simple configuration.

A behavior monitoring method of the present disclosure is summarized as a behavior monitoring method of monitoring a behavior of a monitoring target person living in a house, the behavior monitoring method including detecting the monitoring target person by using multiple detection units in the house, acquiring a distance between two points among the multiple detection units in advance to store the distance in a storage section, and storing reaction time as reaction data in the storage section in a case where any of the multiple detection units reacts, and calculating a movement distance of the monitoring target person per unit time based on data stored in the storage section.

In the behavior monitoring method of the present disclosure, since the movement distance of the monitoring target person per unit time is calculated based on reaction data (reaction time) of the multiple detection units, the behavior of the monitoring target person can be monitored appropriately with a simple configuration. In addition, by using a detection unit provided in a house, such as a light switch, as the detection unit, it is possible to monitor the behavior of the monitoring target person without newly installing a sensor.

### Brief Description of Drawings

Fig. 1 is a schematic configuration diagram of a behavior monitoring system according to the present embodiment.
Fig. 2 is a diagram illustrating an example of a sensor installed in each room of a residence.
Fig. 3 is a flowchart illustrating an example of data measurement processing.
Fig. 4 is a flowchart illustrating an example of data reception processing.
Fig. 5 is a flowchart illustrating an example of behavior determination processing.
Fig. 6 is a diagram illustrating an example of a two-point distance table.
Fig. 7 is a diagram illustrating a movement distance and a staying time in each room as a graph.
Fig. 8 is a diagram illustrating a state in which a room in which a monitoring target person is present is estimated using a monitoring camera.
Fig. 9 is a diagram illustrating the state in which the room in which the monitoring target person is present is estimated using the monitoring camera.
Fig. 10 is a diagram illustrating a state in which a room in which the monitoring target person is present is estimated using two occupancy sensors.

### Description of Embodiments

Next, an embodiment of the present disclosure will be described with reference to the drawings.

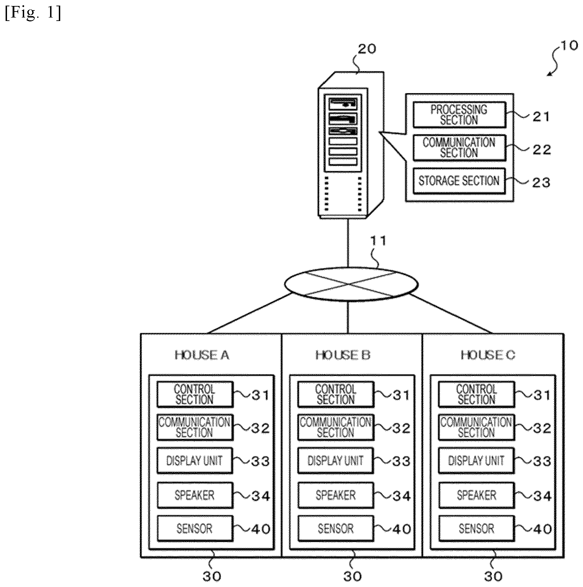

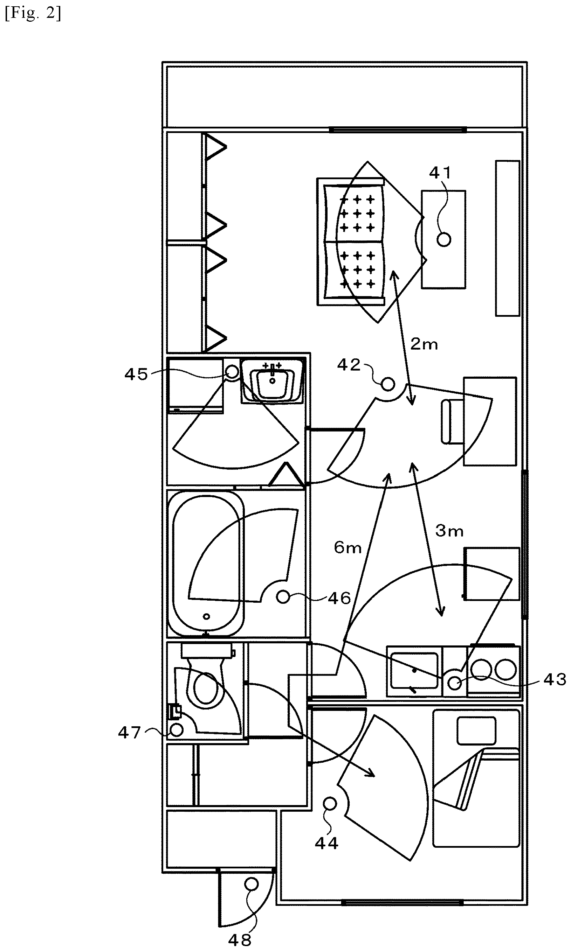

Fig. 1 is a schematic configuration diagram of behavior monitoring system 10 of the present embodiment. As illustrated in Fig. 1, behavior monitoring system 10 of the present embodiment includes management server 20 that manages the entire system, and monitoring apparatuses 30 respectively installed in residences A to C in which a monitoring target person lives. Residences A to C are residences in which, for example, an elderly person or a person requiring care lives alone, and include, for example, an L (living room) D (dining room) K (kitchen) room, a bedroom, a washroom, a bathroom, a toilet room, and an entrance as illustrated in Fig. 2. Behavior monitoring system 10 can be used, for example, to monitor a behavior of an elderly person or a person requiring care as the monitoring target person in place of a caregiver and to find an abnormality in the behavior at an early stage.

Monitoring apparatus 30 includes control section 31, communication section 32, display unit 33, speaker 34, and sensors 40. Control section 31 is configured as a microprocessor including CPU as a main component, and includes ROM, RAM, and the like in addition to the CPU. Display unit 33 and speaker 34 output various information from management server 20 through display or audio. In the present embodiment, display unit 33 is configured as a touch panel type display unit with which an operator can input.

As illustrated in Fig. 2, sensors 40 are sensors for detecting where the monitoring target person who lives in the residence is, and include occupancy sensors 41, 42, 43, 44, 45, 46, and 47 provided in each room, and door sensor 48 provided on an entrance door.

Occupancy sensors 41 to 47 are sensors that detect a person in a detection area in a non-contact manner, and are configured as, for example, infrared sensors that sense an infrared ray and convert the infrared ray into an electric signal. Occupancy sensors 41, 42, and 43 are provided in the living room, dining room, and kitchen of the LDK room, respectively. Occupancy sensor 44 is provided in the bedroom, and occupancy sensor 45 is provided in the washroom. Occupancy sensor 46 is provided in the bathroom, and occupancy sensor 47 is provided in the toilet room.

Door sensor 48 detects opening and closing of the entrance door and is, for example, a magnet-type opening/closing sensor including a permanent magnet fixed to a door side and a magnetic sensor fixed to a frame side.

Management server 20 includes processing section 21, communication section 22, and storage section 23. Processing section 21 is configured as a microprocessor including CPU as a main component, and includes ROM, RAM, and the like in addition to the CPU. Communication section 22 of management server 20 is connected to communication section 32 of each monitoring apparatus 30 via network 11 such as the Internet, and management server 20 and each monitoring apparatus 30 exchange data and a signal with each other via communication sections 22 and 32. Storage section 23 is configured with an HDD, an SSD, or the like, receives data measured by each monitoring apparatus 30, and stores the data for a certain period of time.

Next, an operation of the behavior monitoring system configured as described above, that is, an operation of each monitoring apparatus 30 and an operation of management server 20 will be described. The operation of each monitoring apparatus 30 includes data measurement processing. The operation of management server 20 includes data reception processing and behavior determination processing.

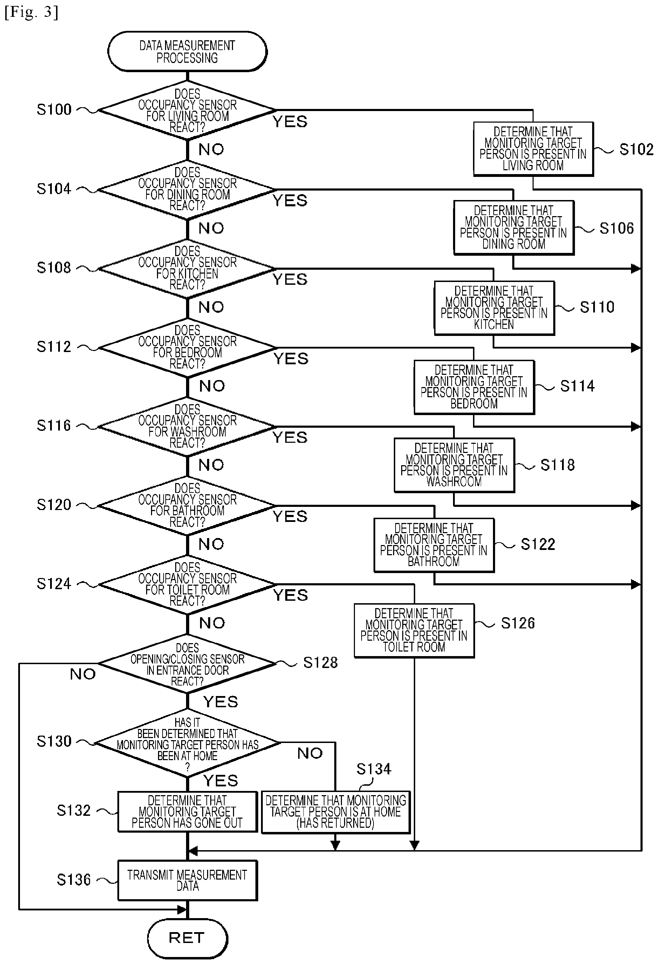

The data measurement processing is processing of measuring (collecting) a location of the monitoring target person from a sensor provided in each room of the residence. Fig. 3 is a flowchart illustrating an example of data measurement processing executed by control section 31 of each monitoring apparatus 30. This processing is repeatedly executed at a predetermined time interval.

When the data measurement processing is executed, control section 31 of monitoring apparatus 30 first determines whether occupancy sensor 41 for the living room provided in the living room reacts (step S100). When it is determined that occupancy sensor 41 for the living room reacts, control section 31 determines that the monitoring target person is present in the living room (step S102), transmits the determination result as measurement data to management server 20 (step S136), and ends the data measurement processing.

When it is determined in step S100 that occupancy sensor 41 for the living room does not react, control section 31 subsequently determines whether occupancy sensor 42 for the dining room provided in the dining room reacts (step S104). When it is determined that occupancy sensor 41 for the dining room reacts, control section 31 determines that the monitoring target person is present in the dining room (step S106), transmits the determination result as measurement data to management server 20 (step S136), and ends the data measurement processing.

When it is determined in step S104 that occupancy sensor 42 for the dining room does not react, control section 31 subsequently determines whether occupancy sensor 43 for the kitchen provided in the kitchen reacts (step S108). When it is determined that occupancy sensor 43 for the kitchen reacts, control section 31 determines that the monitoring target person is present in the kitchen (step S110), transmits the determination result as measurement data to management server 20 (step S136), and ends the data measurement processing.

When it is determined in step S108 that occupancy sensor 43 for the kitchen does not react, control section 31 subsequently determines whether occupancy sensor 44 for the bedroom provided in the bedroom reacts (step S112). When it is determined that occupancy sensor 44 for the bedroom reacts, control section 31 determines that the monitoring target person is present in the bedroom (step S114), transmits the determination result as measurement data to management server 20 (step S136), and ends the data measurement processing.

When it is determined in step S112 that occupancy sensor 44 for the bedroom does not react, control section 31 subsequently determines whether occupancy sensor 45 for the washroom provided in the washroom reacts (step S116). When it is determined that occupancy sensor 45 for the washroom reacts, control section 31 determines that the monitoring target person is present in the washroom (step S118), transmits the determination result as measurement data to management server 20 (step S136), and ends the data measurement processing.

When it is determined in step S116 that occupancy sensor 45 for the washroom does not react, control section 31 subsequently determines whether occupancy sensor 46 for the bathroom provided in the bathroom reacts (step S120). When it is determined that occupancy sensor 46 for the bathroom reacts, control section 31 determines that the monitoring target person is present in the bathroom (step S122), transmits the determination result as measurement data to management server 20 (step S136), and ends the data measurement processing.

When it is determined in step S120 that occupancy sensor 46 for the bathroom does not react, control section 31 subsequently determines whether occupancy sensor 47 for the toilet room provided in the toilet room reacts (step S124). When it is determined that occupancy sensor 47 for the toilet room reacts, control section 31 determines that the monitoring target person is present in the toilet room (step S126), transmits the determination result as measurement data to management server 20 (step S136), and ends the data measurement processing.

When it is determined in step S124 that occupancy sensor 47 for the toilet room does not react, control section 31 subsequently determines whether door sensor 48 for the entrance provided in the entrance door reacts (step S128). When it is determined that door sensor 48 for the entrance reacts, control section 31 determines whether it has been determined in step S134 described later that the monitoring target person has been at home (step S130). When it is determined that it has been determined that the monitoring target person has been at home, control section 31 determines that the monitoring target person has gone out (step S132), transmits the determination result as measurement data to management server 20 (step S136), and ends the data measurement processing. On the other hand, when it is determined that it has not been determined that the monitoring target person has been at home (it is determined that the monitoring target person has been out), control section 31 determines that the monitoring target person has returned, that is, is at home (step S134), transmits the determination result as measurement data to management server 20 (step S136), and ends the data measurement processing.

Next, operations (the data reception processing and the behavior determination processing) of management server 20 will be described.

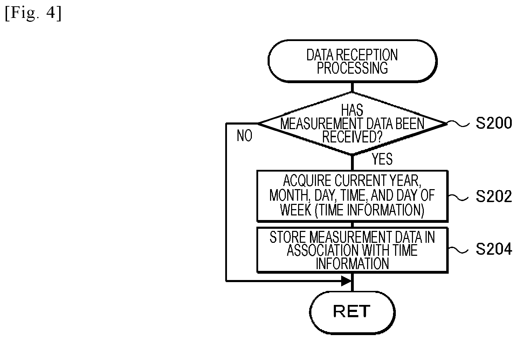

The data reception processing is processing of receiving measurement data transmitted from each monitoring apparatus 30. Fig. 4 is a flowchart illustrating an example of data reception processing executed by processing section 21 of management server 20. This processing is repeatedly executed at a predetermined time interval.

When the data reception processing is executed, processing section 21 of management server 20 first determines whether measurement data has been received from monitoring apparatus 30 (step S200). When it is determined that measurement data has not been received, processing section 21 ends the data reception processing. On the other hand, when it is determined that measurement data has been received, processing section 21 accesses a time server via the Internet to acquire the current year, month, day, time (hour/minute/second), and day of the week as time information (step S202), stores the acquired time information in storage section 23 in association with the received measurement data (step S204), and ends the data reception processing. The time information may be acquired by reading the current time from a real-time clock (RTC).

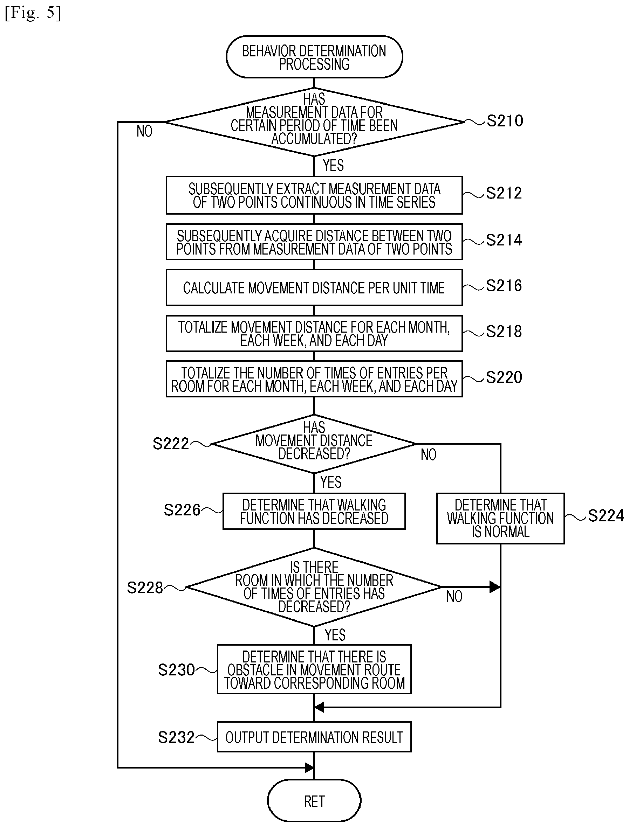

In the behavior determination processing, it is determined whether a behavior of the monitoring target person is appropriate based on the measurement data collected from each monitoring apparatus 30. Fig. 5 is a flowchart illustrating an example of the behavior determination processing executed by processing section 21 of management server 20. This processing is repeatedly executed at a predetermined time interval.

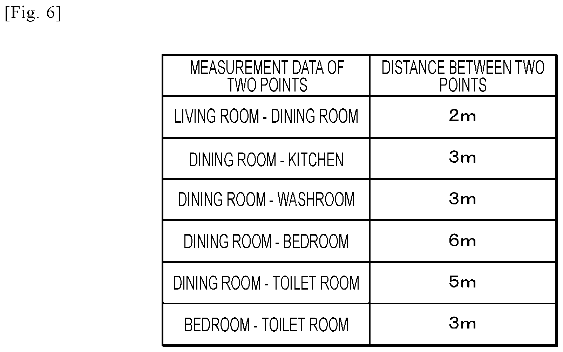

When the behavior determination processing is executed, processing section 21 first determines whether measurement data (data of a room in which the monitoring target person has been present) for a certain period of time (for example, for one month or for one week) has been accumulated in storage section 23 (step S210). When it is determined that the measurement data for the certain period of time has not accumulated, processing section 21 ends the behavior determination processing. On the other hand, when it is determined that the measurement data for the certain period of time has been accumulated, processing section 21 subsequently extracts, from the measurement data for the certain period of time, pieces of measurement data of two points that are continuous in time series and have different room determination (step S212), and subsequently acquires distances between two points based on the pieces of measurement data of the two points subsequently extracted (step S214). The distances between two points are acquired by obtaining a relationship between measurement data of two points and distances between two points in advance, registering the relationship in storage section 23 as a two-point distance table, and deriving a corresponding distance between two points from the two-point distance table when the measurement data of the two points is given. Fig. 6 shows an example of the two-point distance table.

After subsequently acquiring the distances between two points, processing section 21 calculates an integrated value of the acquired distances between two points per unit time (for example, per 30 minutes or per hour) to calculate a movement distance of the monitoring target person per unit time (step S216). Subsequently, processing section 21 totalizes the calculated movement distance per unit time for each month, each week, and each day (step S218), and totalizes the number of times of entries per room (per area) for each month, each week, and each day (step S220). Next, processing section 21 compares the calculated movement distance with a past calculated value to determine whether the movement distance of the monitoring target person has decreased by a predetermined degree or more (step S222). This determination may be made by, for example, comparing a value of the current day with a value of the last day, comparing a value of the current week with a value of the last week, or comparing a value of the current month with a value of the last month. Alternatively, an evaluation value such as an average value of the totalized values may be set, and the set evaluation value may be compared with a past evaluation value to make the determination. When it is determined that the movement distance of the monitoring target person has not decreased by the predetermined degree or more, processing section 21 determines that a walking function of the monitoring target person is normal (step S224), transmits the determination result (normal) to monitoring apparatus 30 (step S232), and ends the behavior determination processing. Monitoring apparatus 30 outputs information indicating that a current state is normal through display unit 33 or speaker 34 based on the received determination result.

On the other hand, when it is determined that the movement distance of the monitoring target person has decreased by the predetermined degree or more, processing section 21 determines that the walking function of the monitoring target person has decreased (step S226), and further compares the number of times of entries totalized for each room with a past value to determine whether there is a room in which the number of times of entries has decreased by a predetermined degree or more (step S228). This determination may be made for each room by, for example, comparing a value of the current day with a value of the last day, comparing a value of the current week with a value of the last week, or comparing a value of the current month with a value of the last month. When it is determined that there is a corresponding room, processing section 21 determines that there is an obstacle in a movement route toward the corresponding room (step S230), transmits the determination result that the walking function has decreased and there is an obstacle in the movement route toward the corresponding room to monitoring apparatus 30 (step S232), and ends the behavior determination processing. On the other hand, when it is determined in step S228 that there is no corresponding room, processing section 21 transmits the determination result that the walking function has decreased to monitoring apparatus 30 (step S232), and ends the behavior determination processing. Monitoring apparatus 30 outputs information indicating that the current state is abnormal and the content thereof through display unit 33 and speaker 34 based on the received determination result. Accordingly, the monitoring target person can obtain information indicating that the walking function has decreased, which contributes to early treatment. Furthermore, by combining with the presence or absence of a decrease in the number of times of entries, it is possible to confirm where an influence due to a decrease in the walking function appears in life.

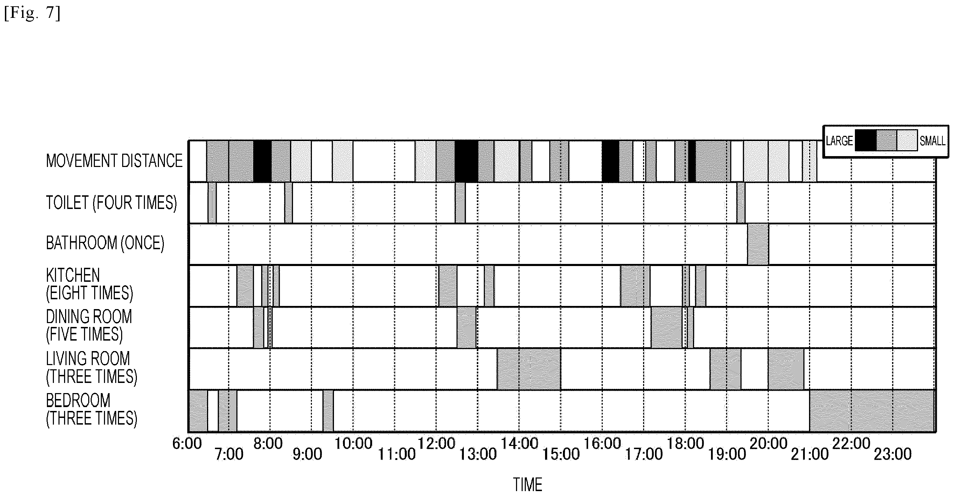

Processing section 21 may transmit the movement distance per unit time, the number of times of entries into each room, and a staying time in each room to monitoring apparatus 30, so that monitoring apparatus 30 displays a graph of data thereof on display unit 33 (see Fig. 7). In addition, processing section 21 may transmit the determination result to a portable information terminal such as a smartphone registered in advance in order to notify the monitoring target person, a protector of the monitoring target person, or the like of the determination result.

Here, a correspondence relationship between main elements of the embodiment and main elements of the present disclosure described in Claims will be described. That is, occupancy sensors 41 to 47 of the present embodiment correspond to occupancy sensors of the present disclosure, storage section 23 corresponds to a storage section, and processing section 21 corresponds to a processing section. Display unit 33 corresponds to an output section. Occupancy sensors 41 to 47 and door sensor 48 correspond to a detection unit.

It is needless to say that the present disclosure is not limited in any way to the above-described embodiments, and the present disclosure can be embodied in various aspects as long as the aspects fall within the technical scope of the present disclosure.

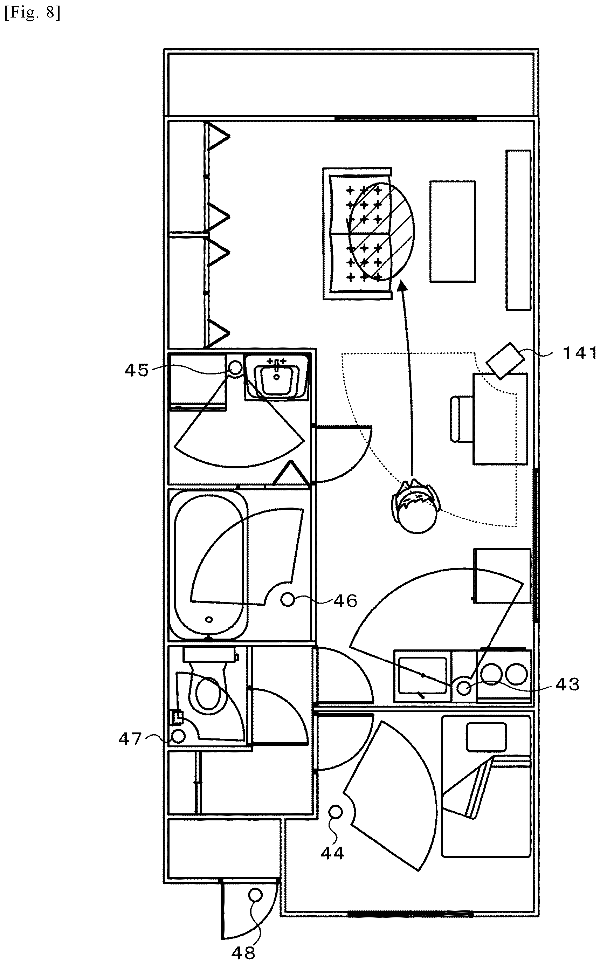

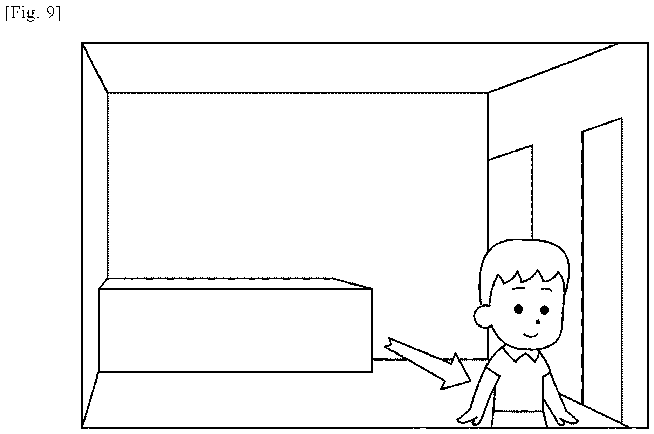

For example, in the above-described embodiment, processing section 21 estimates a room (area) in which the monitoring target person is present based on detection signals from occupancy sensors 41 to 47 provided in the rooms of the house. However, as illustrated in Fig. 8, monitoring camera 141 may be installed in some rooms (areas) instead of the occupancy sensor, and processing section 21 may estimate a room (area) in which the monitoring target person is present based on an imaging signal from monitoring camera 141. In this case, when the monitoring target person has moved from an inside of an imaging area of monitoring camera 141 to an outside of the imaging area, a direction in which the monitoring target person has moved in the imaging area may be recognized from a captured image to estimate a room (area) as a destination of the monitoring target person. For example, as illustrated in Figs. 8 and 9, when the monitoring target person is imaged by monitoring camera 141 having the dining room as the imaging area and it is recognized in the captured image that the monitoring target person has moved in a direction toward the living room, processing section 21 can estimate that the monitoring target person is present in the living room.

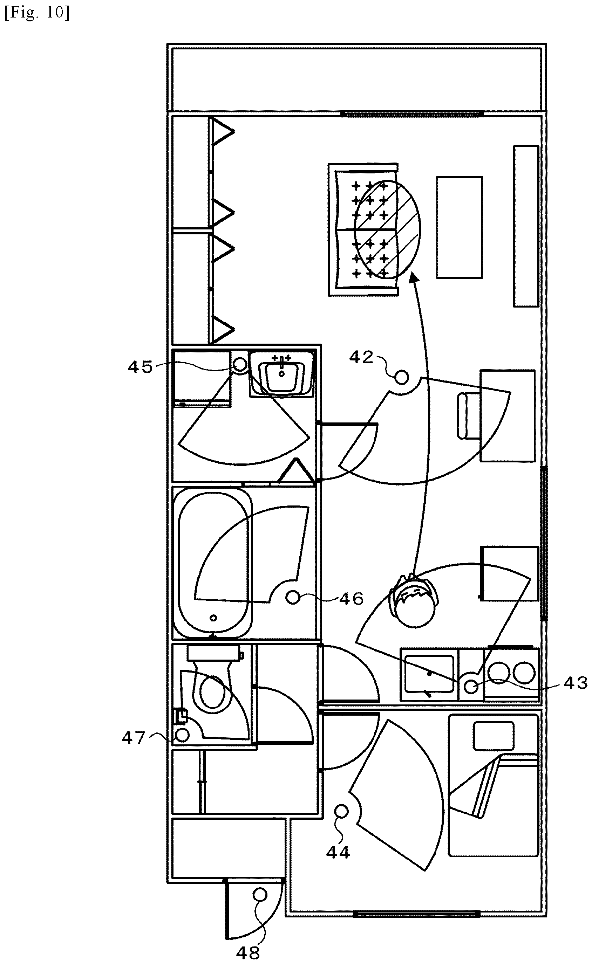

In addition, processing section 21 may estimate an area in which the monitoring target person is present by using two occupancy sensors having areas adjacent to each other as detection areas even in a case where two occupancy sensors do not react. For example, as illustrated in Fig. 10, processing section 21 can estimate that the monitoring target person is present in the living room in a case where, in the LDK room, a state of occupancy sensor 42 provided in the dining room changes from a non-reacting state to a reacting state, and then to the non-reacting state after a state of occupancy sensor 43 provided in the kitchen changes from a non-reacting state to a reacting state, and then to the non-reacting state. This makes it possible to reduce the number of occupancy sensors.

Further, processing section 21 may detect the monitoring target person using multiple detection units provided in advance in the house. For example, the monitoring target person may be detected based on signals from multiple light switches provided for each room in order to turn on and off a light of the room. In this case, processing section 21 may register distances between rooms in advance, and acquire a corresponding distance among the registered distances between rooms as a movement distance when a light switch of one room is turned off from on and then a light switch of another room is turned on from off.

In the behavior monitoring system of the present disclosure described above, a processing section acquires a distance between two points among the multiple occupancy sensors in advance and stores the distance between two points in the storage section. Then, the processing section stores the reaction time as the reaction data in the storage section in a case where any of the multiple occupancy sensors reacts, and calculates the movement distance of the monitoring target person per unit time based on the data stored in the storage section. Since the occupancy sensor can detect the monitoring target person in a non-contact manner, the occupancy sensor has a degree of freedom of installation and can be installed at a position that does not interfere with the behavior of the monitoring target person. Further, since the movement distance of the monitoring target person per unit time is calculated based on reaction data (reaction time) of each occupancy sensor, the behavior of the monitoring target person can be monitored appropriately with a simple configuration.

In the behavior monitoring system of the present disclosure as above, the processing section may further compare an increase or decrease in the movement distance per unit time in time series. In this way, it is possible to accurately determine a decrease in the walking function of the monitoring target person with a simple configuration. In this case, an output section that outputs a warning in a case where the movement distance per unit time decreases by a predetermined distance or more in time series may be provided.

In the behavior monitoring system of the present disclosure, the multiple occupancy sensors may be respectively provided in multiple rooms of the house, and the processing section may count the number of times of entries of the monitoring target person into each room and store the number of times of entries in the storage section in a case where any of the multiple occupancy sensors reacts. This makes it possible to appropriately obtain information on a change in the behavior of the monitoring target person. In this case, the processing section may further compare an increase or decrease in the number of times of entries into each room in time series. In this case, an output section that outputs a warning in a case where the number of times of entries into the room decreases by a predetermined number of times or more in time series may be provided.

Further, the present disclosure is not limited to a form of a behavior monitoring system, and may be a form of a behavior monitoring method. In this case, a monitoring target person may be detected by using multiple detection units in the house, a distance between two points among the multiple detection units may be acquired in advance and stored in a storage section, reaction time may be stored as reaction data in the storage section in a case where any of the multiple detection units reacts, and a movement distance of the monitoring target person per unit time may be calculated based on data stored in the storage section.

### Industrial Applicability

The present disclosure can be used in a manufacturing industry of a behavior monitoring system or the like.

### Reference Signs List

10 behavior monitoring system, 11 network, 20 management server, 21 processing section, 22 communication section, 23 storage section, 30 monitoring apparatus, 31 control section, 32 communication section, 33 display unit, 34 speaker, 40 sensor, 41 to 47 occupancy sensor, 48 door sensor, 141 monitoring camera

## Claims

1. A behavior monitoring system configured to monitor a behavior of a monitoring target person living in a house, the behavior monitoring system comprising:
a detection section installed in the house and including multiple occupancy sensors configured to detect the monitoring target person in a non-contact manner in detection ranges different with each other;
a storage section configured to store data; and
a processing section configured to acquire a distance between two points among the multiple occupancy sensors in advance to store the distance in the storage section, store reaction time as reaction data in the storage section in a case where any of the multiple occupancy sensors reacts, and calculate a movement distance of the monitoring target person per unit time based on data stored in the storage section.

2. The behavior monitoring system according to claim 1, wherein
the processing section is further configured to compare an increase or decrease in the movement distance per unit time in time series.

3. The behavior monitoring system according to claim 2, further comprising:
an output section configured to output a warning in a case where the movement distance per unit time decreases by a predetermined distance or more in time series.

4. The behavior monitoring system according to any one of claims 1 to 3, wherein
the multiple occupancy sensors are respectively provided in multiple rooms of the house, and
the processing section in configured to, in a case where any of the multiple occupancy sensors reacts, counts the number of times of entries of the monitoring target person into each room to store the number of times of entries in the storage section .

5. The behavior monitoring system according to claim 4, wherein
the processing section is further configured to compare an increase or decrease in the number of times of entries into each room in time series.

6. The behavior monitoring system according to claim 5, further comprising:
an output section configured to output a warning in a case where the number of times of entries into the room decreases by a predetermined number of times or more in time series.

7. A behavior monitoring method of monitoring a behavior of a monitoring target person living in a house, the behavior monitoring method comprising:
detecting the monitoring target person by using multiple detection units in the house;
acquiring a distance between two points among the multiple detection units in advance to store the distance in a storage section; and
storing reaction time as reaction data in the storage section in a case where any of the multiple detection units reacts, and calculating a movement distance of the monitoring target person per unit time based on data stored in the storage section.
